(19) Europäisches Patentamt
European Patent Office
Office européen des brevets

(11) Veröffentlichungsnummer: **0 270 489 B1**

(12) **EUROPÄISCHE PATENTSCHRIFT**

(45) Veröffentlichungstag der Patentschrift: **17.04.91**

(51) Int. Cl.5: **C09B 57/00**, C11D 3/40

(21) Anmeldenummer: **87810627.7**

(22) Anmeldetag: **02.11.87**

# (54) Anionische Cyclo-diyliden Verbindungen, deren Herstellung und Verwendung in Waschmitteln als Nuancierfarbstoffe.

(30) Priorität: **07.11.86 CH 4454/86**

(43) Veröffentlichungstag der Anmeldung:
**08.06.88 Patentblatt 88/23**

(45) Bekanntmachung des Hinweises auf die Patenterteilung:
**17.04.91 Patentblatt 91/16**

(84) Benannte Vertragsstaaten:
**CH DE ES FR GB IT LI**

(56) Entgegenhaltungen:

**CHEMICAL ABSTRACTS, Band 104, Nr. 5, 3. Februar 1986, Seite 538, Zusammenfassung Nr. 33972n, Columbus, Ohio, US; B.P. BESPALOV et al.: "Reaction of gamma-pyrones with 1,3-bis(dicyanomethylene)indan", & KHIM. GETEROTSIKL. SOEDIN. 1985, (5), 603-8**

**CHEMICAL ABSTRACTS, Band 79, Nr. 15, 15. Oktober 1973, Seite 403, Zusammenfassung Nr. 91816r, Columbus, Ohio, US; P. PASTORS et al.: "Condensation of dicarbonyl compounds with malonitrile. V. Reactions between 1,1,3,3-tetracyanoindandimethane and aromatic aldehydes", & LATV. PSR ZINAT. AKAD. VESTIS, KIM. SER. 1973, (3), 355-7**

**CHEMICAL ABSTRACTS, Band 77, Nr. 17, 23. Oktober 1972, Seite 422, Zusammenfassung Nr. 114095e, Columbus, Ohio, US; E. GUDRINIECE et al.: "Condensation of 1,3-indandione with malononitrile", & DOKL. AKAD. NAUK SSR 1972, 204(4), 874-5**

(73) Patentinhaber: **CIBA-GEIGY AG**
**Klybeckstrasse 141**
**CH-4002 Basel(CH)**

(72) Erfinder: **Naef, Rudolf, Dr.**
**Im Budler 6**
**CH-4419 Lupsingen(CH)**
Erfinder: **Eckhardt, Claude, Dr.**
**16, Rue des Jonquilles**
**F-68400 Riedisheim(FR)**

**EP 0 270 489 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue anionische Cyclo-diyliden Verbindungen der Formel

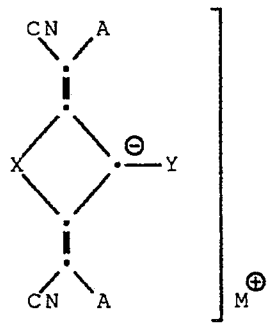

(I)

worin
X ein substituierter oder unsubstituierter, 5- oder 6-gliedriger monocyclischer oder polycyclischer aromatischer oder heteroaromatischer Rest ist,
Y Wasserstoff oder ein substituierter oder unsubstituierter, Alkenyl-, Alkyl- oder Cycloalkyl-Rest mit 1 bis 6 Kohlenstoffatomen oder ein 5- oder 6-gliedriger monocyclischer oder polycyclischer aromatischer oder heteroaromatischer Rest,
A, CN oder eine Carbonylverbindung ist,
M ein Alkalimetall- oder Ammoniumion ist, mit der Bedingung, dass X kein unsubstituiertes Phenylen ist, wenn Y Wasserstoff oder unsubstituiertes Phenyl ist.

Bei dem Rest X handelt es sich beispielsweise um einen vicinal oder periständig gebundenen Pyrrol-, Pyrazol-, Imidazol-, Furan-, Thiophen-, Benzol-, Pyridin-, Pyridazin-, Pyrimidin-, Pyrazin-, Indolizin-, Indol-, Isoindol-, Benzofuran-, Benzothiophen-, Benzothiazol-, Benzoxazol-, Naphthalin-, Chinolin-, Isochinolin-, Cinnolin-, Phthalazin-, Chinazolin-, Chinoxalin-, Anthracen-, Acridin-, Phenanthren- und Phenanthridin-Rest in einer der möglichen stellungsisomeren Formen.

Bevorzugt ist X ein Pyridin-, Pyridazin-, Pyrimidin-, Pyrazin-, Naphthalin-, Chinolin-, Benzothiazol-, Benzoxazol- oder ein Benzolrest, insbesondere der unsubstituierte Benzolrest.

Y in der Bedeutung von substituiertem oder unsubstituiertem Alkenyl, Alkyl oder Cycloalkyl mit 1 bis 6 Kohlenstoffatomen, stellt beispielsweise dar: Methyl, Ethyl, n-Propyl, n-Butyl, n-Pentyl, n-Hexyl und die entsprechenden isomeren Reste sowie Ethenyl, Cyclopentyl, Cyclohexyl, Cyclopentenyl, Cyclohexenyl. Stellt Y einen 5- oder 6-gliedrigen monocyclischen oder polycyclischen aromatischen oder heteroaromatischen Rest dar, so kommen die weiter oben für X beschriebenen Reste in Frage, wobei die oben für X bevorzugt verwendeten Reste auch für Y bevorzugt sind.

Der Rest Y kann dabei gleich oder ungleich X sein, mit der Einschränkung, dass X kein unsubstituiertes Phenylen sein darf, wenn Y Wasserstoff oder unsubstituiertes Phenyl ist.

Insbesondere steht für Y der substituierte Phenylrest.

Die Reste X und Y können unabhängig voneinander einfach oder mehrfach und wenn mehrfach dann gemischt oder ungemischt substituiert sein, wobei als Substituenten beispielsweise in Frage kommen: Phenyl-, Alkyl- oder Cycloalkylgruppen mit 1 bis 6 Kohlenstoffatomen, Halogen, SR, $SO_3R$, $NO_2$, NHR, $NR_2$, NRCOR, OR, OCOR, CHO, COR, COOR, $CONR_2$, CN. Die Phenyl-, Alkyl- und Cycloalkylsubstituenten können ihrerseits mit den genannten Substituenten substituiert sein. In den genannten Substituenten bedeutet R Wasserstoff oder ein Phenyl-, Alkyl oder Cycloalkylrest mit 1 bis 6 Kohlenstoffatomen.

Bedeutet A eine Carbonylverbindung

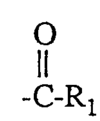,

so handelt es sich beim Rest $R_1$ beispielsweise um Wasserstoff oder Phenyl-, Alkyl- oder Cycloalkyl mit 1 bis 6 Kohlenstoffatomen, der gegebenenfalls seinerseits mit Resten R" z.B. Halogen, SR', $SO_3R'$, $NO_2$, NHR', $NR_2'$, NR'COR', OR', OCOR', CHO, $COR_1$, COOR', $CONR_2'$, CN oder Alkyl mit 1 bis 6 Kohlenstoffatomen substituiert ist, wobei R' Wasserstoff oder ein Phenyl-, Alkyl- oder Cycloalkylrest mit 1 bis 6

Kohlenstoffatomen bedeutet, oder $R_1$ ist gleich R".

Bevorzugt steht für A die Nitrilgruppe.

Das Kation M stellt ein Alkalimetall-ion oder ein Ammoniumion dar, zum Beispiel Lithium, Natrium, oder Kalium oder Tetra-$C_1$-$C_6$-alkylammonium, wie Tetramethylammonium.

Bei den Verbindungen der Formel I handelt es sich beispielsweise um bläuliche bis violette Verbindungen die wasserlöslich sind und am Tageslicht während 8 bis 32 Stunden ausbleichen.

In den bevorzugten Verbindungen der Formel I bedeutet X und Y je einen Pyridin-, Pyridazin-, Pyrimidin-, Pyrazin-, Naphthalin-, Chinolin-, Benzothiazol-, Benzoxazol- oder Benzolrest, A bedeutet CN und M stellt das Lithium-, Natrium-, Kalium- oder Ammoniumion dar. Von besonderem Interesse ist vor allem diejenige Verbindung, worin X unsubstituiertes Phenylen, Y substituiertes Phenyl, A den Cyanrest und M das Natriumion bedeutet.

Ein weiterer Gegenstand der Anmeldung ist eine Verbindung der Formel

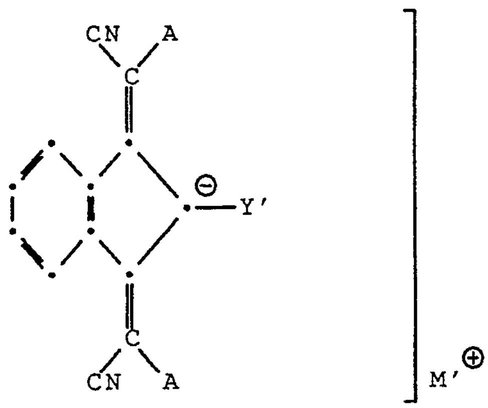

(Ia)

worin Y' Wasserstoff oder Phenyl und M' ein Alkalimetallion vorzugsweise Natrium bedeutet. Besonders bevorzugt ist die Verbindung der Formel

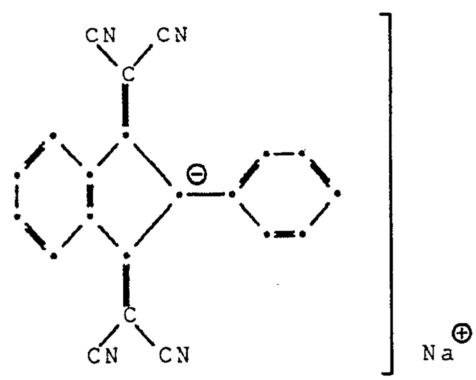

(Ib).

Diese Verbindung zeichnet sich durch eine überraschenderweise gute Wasserlöslichkeit sowie eine zeitlich begrenzte Lichtbeständigkeit aus.

Die Herstellung der erfindungsgemässen Verbindungen der Formeln I erfolgt durch Umsetzung von Verbindungen der Formel

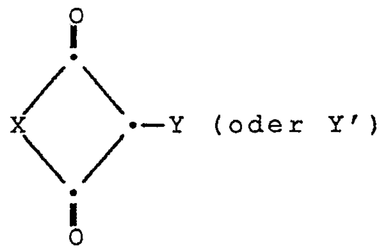

(II)

mit Verbindungen der Formel

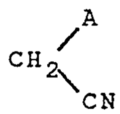

(III),

worin X, Y bzw. Y' und A die weiter oben angegebene Bedeutung haben, in einem protischen Lösungsmittel, wie zum Beispiel Alkohole, Glykole, Polyole, Glykolether oder auch in Ethern oder Benzol oder in Gemischen von diesen, und in Gegenwart eines sauren, basischen oder amphoteren Katalysators wie z.B. einer anorganischen oder organischen Säure oder Base, oder einem Salz oder Mischung von diesen, wobei insbesondere ein sekundäres oder tertiäres Amin, Metallalkoholat, Metallhydroxid, Ammoniumsalz wie Ammoniumacetat oder Pyridin verwendet wird.

Die Aufarbeitung erfolgt in an und für sich bekannter Weise, zum Beispiel durch Ausfällen mit Alkali- oder Ammoniumsalzen wie Tetra-$C_1$-$C_6$-alkylammoniumhydroxid und anschliessender Filtration.

Die Umsetzung wird in einem Temperaturbereich von 10 °C bis zur Siedetemperatur des jeweiligen Lösungsmittels bzw. Gemisches durchgeführt.

Die Verbindungen der Formel II und III sind bekannt oder können nach bekannten Methoden hergestellt werden.

Die Erfindung betrifft des weiteren die Verwendung der Verbindungen der Formel Ic

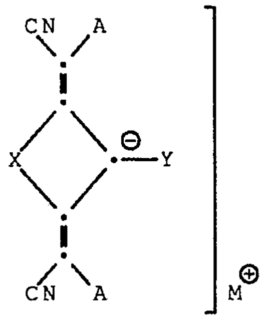

(Ic),

worin

X ein substituierter oder unsubstituierter, 5- oder 6-gliedriger monocyclischer oder polycyclischer aromatischer oder heteroaromatischer Rest ist,

Y Wasserstoff oder ein substituierter oder unsubstituierter, Alkenyl-, Alkyl- oder Cycloalkyl-Rest mit 1 bis 6 Kohlenstoffatomen oder ein 5- oder 6-gliedriger monocyclischer oder polycyclischer aromatischer oder heteroaromatischer Rest ist,

M ein Alkalimetall- oder Ammoniumion ist,

A, CN oder eine Carbonylverbindung

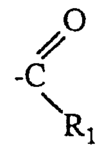

ist, worin $R_1$ beispielsweise Wasserstoff oder ein Phenyl-, Alkyl- oder Cycloalkylrest mit 1 bis 6 Kohlenstoffatomen ist, der gegebenenfalls seinerseits mit Resten R" gleich Halogen, SR', $SO_3R'$, $NO_2$, NHR', $NR_2'$, NR'COR', OR', OCOR', CHO, $COR_1$, COOR', $CONR_2'$, CN oder Alkyl mit 1 bis 6 Kohlenstoffatomen substituiert ist, wobei R' Wasserstoff oder ein Phenyl-, Alkyl- oder Cycloalkylrest mit 1 bis 6 Kohlenstoffatomen bedeutet, oder $R_1$ ist gleich R", als Nuancierfarbstoffe.

Sie können einem handelsüblichen festen oder flüssigen Waschmittel mit kationischem, anionischem oder neutralem Charakter zugesetzt werden. Die Nuancierfarbstoffmenge liegt zwischen 0,0025 bis 0,01% bezogen auf die Waschmittelmenge. Gegebenenfalls wird dem Waschmittel noch ein Lichtschutzmittel zugefügt.

Insbesonders verleiht die Verbindung der Formel

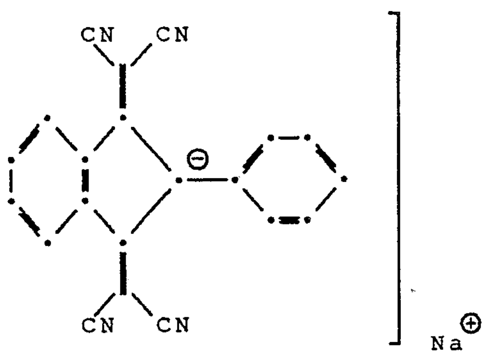

(Ib)

dem Waschgut eine brilliante bläuliche Nuance.

Es ist auch möglich verschiedene Verbindungen der Formel Ic zu mischen oder die Verbindungen der Formel Ic oder ein Gemisch dieser Verbindungen mit bekannten Nuancierfarbstoffen, Bleichmitteln und/oder optischen Aufhellern vermengt einzusetzen.

Die Nuancierfarbstoffe der Formel Ic oder deren genannte Gemische werden insbesondere Waschmitteln, zum Waschen von Textilien aus Cellulose oder Cellulose/Polyester Mischmaterial, zugegeben.

Die Vorteile der Verbindungen der Formel Ic für die Verwendung als Nuancierfarbstoffe gegenüber bekannten Nuancierfarbstoffen, liegen in der Verbesserung des optischen Effektes verbunden mit einem guten Aufziehvermögen, vor allem geringer Akkumulation bei wiederholten Waschbehandlungen. Ausserdem sind die oben genannten Verbindungen sprühbeständig und besitzen eine gute Stabilität in wässriger Lösung.

In den nachfolgenden Beispielen, welche die Erfindung näher erläutern, ohne sie darauf zu beschränken, sind Teile- und Prozentangaben immer, soweit nichts anderes angegeben ist, Gewichtsteile und Gewichtsprozente, Schmelz- und Siedepunkte sind, sofern nichts anderes angegeben ist, unkorrigiert.

Beispiel 1: In 100 Teilen 2-Ethoxyethanol werden 13,3 Teile 2-Phenylindan-1,3-dion mit 10 Teilen Malonsäuredinitril unter katalytischer Einwirkung von 2 Teilen Pyridin durch dreitägiges Kochen am Rückfluss zur Reaktion gebracht.

Anschliessend wird das Rohprodukt bei Raumtemperatur mit gesättigter Kochsalzlösung ausgefällt und filtriert. Nach Auflösen des Niederschlags in 200 Teilen Essigsäureethylester, Trocknen der Lösung mit Natriumsulfat und Filtration wird das Filtrat langsam mit Ether versetzt, wodurch 13 Teile reines Produkt der Formel

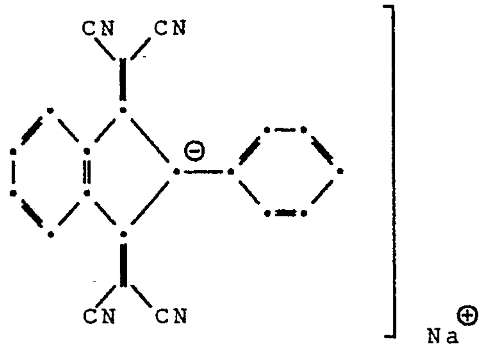

[1]

Smp. >270° ausfallen.

Beispiel 2: Setzt man anstatt 13,3 Teile 2-Phenylindan-1,3-dion, in Beispiel 1 15,3 Teile 2-(4'-Chlorphenyl)-indan-1,3-dion ein, so erhält man die Verbindung der Formel

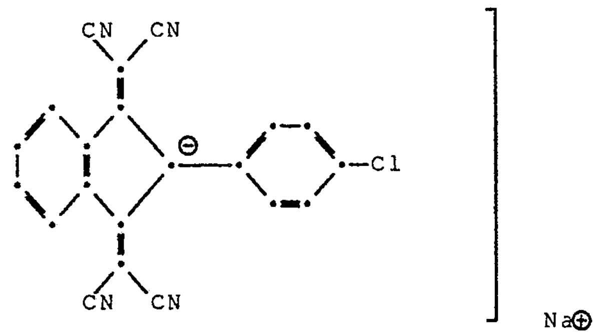

[2] .

Beispiel 3: Zu 100 ml deionisiertem Wasser werden folgende Waschmittel-Komponente unter effizientem Rühren zugegeben:

10,8 g Na-Dodecylbenzolsulfonat
2,2 g Ethoxylierter $C_{15}$-$C_{18}$ Alkohol (mit 9 Ethylenoxid)
3,0 g Seife (aus Behensäure)
30,0g Na-Tripolyphosphat
5,5 g Na-Silikat
1,6 g Carboxymethylcellulose
0,2 g Na-Ethylendiamintetraacetat
37,0 g Na-Sulfat

Nach Homogenisierung dieser Slurry, wird sie in einem Vakuum-Trockenschrank bei 60°C unter 200 Torr während einer Nacht getrocknet. Man erhält somit 100 g einer Masse die durch ein Sieb mit Maschenweite von 0,8 mm gepresst wird. Ein weiteres Sieb mit Maschenweite von 0,315 mm dient zur Eliminierung des feinen, stäubenden Anteils.

Das resultierende, gleichkörnige Waschpulver A wird für Waschversuche verwendet.

Nach dem gleichen Verfahren wird ein Waschpulver B hergestellt, wobei zusätzlich zu den oben beschriebenen Komponenten, noch 0,005 g der gemäss Beispiel 1 hergestellten Verbindung [1] zugegeben wird. Das resultierende Waschpulver B enthält somit 0,005 % dieser blauen Verbindung [1]. Als hellblaues Vergleichs-Waschpulver wird nach dem gleichen Verfahren ein Waschmittel C hergestellt, wobei statt Verbindung [1], 0,005 g des Na-Salzes der Aluminiumphthalocyanin-sulfonsäure eingesetzt wird.

Waschversuche:

3 Stücke à je 10 g Baumwoll-Gewebe, gebleicht und nicht voraufgehellt, werden jeweils in 200 ml Leitungswasser (12°dH) mit 0,8 g des Waschmittels A, bzw. B, bzw. C bei einer Flottentemperatur von 30°C während 15 Minuten gewaschen, dann 30 Sek. unter fliessendem Leitungswasser gespült und nass zur Trocknung an der Leine, im Freien, d.h. unter direkter Einwirkung des Sonnenlichtes, aufgehängt.

Die auf den Geweben angefallene Lichtenergie wird mit einem Pyrheliometer verfolgt (Angaben in Langley). Ca. 2 g Stücke werden aus den Baumwoll-Lappen nach verschiedenen Zeit-Intervallen abgeschnitten, gegebenenfalls im Trockenschrank bei 60 °C im Dunkel fertig getrocknet, und mit einem Spektrophotometer (RFC 3 von Zeiss) nach der Method von Ganz auf Weissgrad und Farbabweichungszahl ( = FAZ) gemessen (Sh. Ganz, Appl. Optics 18(1979), 1073-1078).

Resultate:

Beide Waschmittel B und C verleihen dem Gewebe vor der Belichtung eine im Vergleich zum Waschmittel A leichte, blaustichige Nuance. Die FAZ betragen:

Waschmittel A = 0,5 d.h. praktisch neutral
Waschmittel B = 2,1 d.h. etwas blaustichig
Waschmittel C = 2,2 d.h. etwas blaustichig

Diese Bläuung verschwindet langsam bei C, schneller bei B, was vorteilhaft ist, um in der Praxis bei wiederholten Waschbehandlungen eine zu starke Akkumulation, und zu stärke Tönungen zu vermeiden.

Nach einer Belichtung, im nassen Zustand, bis 15 Langley beträgt die FAZ für Waschmittel B, nur noch 1,5 (eine Spur blaustichig), während mit Waschmittel C eine Belichtung bis zu 170 Langley notwendig ist, um den gleichen FAZ-Wert von 1,5 zu erreichen. Mit Waschmittel A (ohne Bläuungsmittel) bleibt die FAZ auch nach 170 Langley bei 0,5.

Beispiel 4: Die Waschmittel A (= blind) und B (= mit der Verbindung [1]) werden wie im Beispiel 3 vorbereitet. Die Waschversuche werden gemäss den Bedingungen des Beispiels 3 durchgeführt, jedoch mit Trocknung im Dunkel (Trockenschrank).

Nach wiederholter Wäsche bleibt die FAZ mit Waschmittel A bei 0,5 auch nach 10 facher Wäsche unverändert. Mit Waschmittel B steigt die FAZ erwartungsgemäss von 2,1 bis zu 3,7 nach der 5ten Wäsche. Diese bläuliche Nuance nimmt jedoch überraschenderweise nach der fünften

Wäsche nicht mehr zu, und beträgt im Gegenteil 3,2 nach der zehnten Wäsche. Diese begrenzte Akkumulierung der Nuancierung ist von grossem Vorteil, um unerwünschte Verfärbungen in der Praxis zu vermeiden.

Beispiel 5: Die Waschmittel A', B' und C' werden gemäss Beispiel 3 hergestellt, jedoch mit Zugabe von je 0,1 g eines optischen Aufhellers zu allen drei Waschmitteln.

Als optischer Aufheller wird das Na-Salz der Distyrylbiphenyl-disulfonsäure verwendet.

Die mehrfache Wäsche wird wie im Beispiel 3 durchgeführt.

Die Messungen nach Ganz ergaben folgende Werte:

| Anzahl Wäsche / Waschmittel | 1 x | FAZ | 5 x | FAZ | 10 x | FAZ | 20 x | FAZ |
|---|---|---|---|---|---|---|---|---|
| A' = Blind | 179 | - 0,7 | 231 | - 0,4 | 242 | - 0,1 | 246 | 0,3 |
| B' = 0,005 % Verb. [1] | 191 | 0,8 | 248 | 2,0 | 258 | 2,1 | 263 | 2,3 |
| C' = 0,05 % Al PCS | 183 | 0,5 | 237 | 2,5 | 248 | 3,5 | 258 | 4,5 |

Die erreichten Weisseffekte sind auf Grund der blaustichigen Nuancierung mit den Waschmitteln B' und C' besser als mit A'. Dieser Vorteil ist noch deutlicher ausgeprägt bei dem Waschmittel B' (mit der

Verbindung [1]), insbesondere dank des reduzierten Akkumulations-Effektes, und der geringere Nuancenverschiebung nach grünstichigen Tönen (FAZ).

## Ansprüche

1. Anionische Cyclo-diyliden Verbindung der Formel

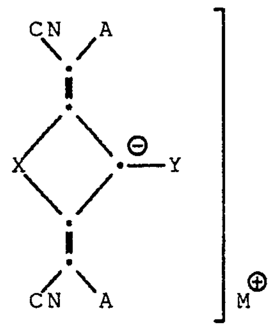

(I)

worin
X ein substituierter oder unsubstituierter, 5- oder 6-gliedriger monocyclischer oder polycyclischer aromatischer oder heteroaromatischer Rest ist,
Y Wasserstoff oder ein substituierter oder unsubstituierter, Alkenyl-, Alkyl- oder Cycloalkyl-Rest mit 1 bis 6 Kohlenstoffatomen oder ein 5- oder 6-gliedriger monocyclischer oder polycyclischer aromatischer oder heteroaromatischer Rest ist,
A, CN oder eine Carbonylverbindung ist,
M ein Alkalimetall- oder Ammoniumion ist, mit der Bedingung, dass X kein unsubstituiertes Phenylen ist, wenn Y Wasserstoff oder unsubstituiertes Phenyl ist.

2. Verbindung gemäss Anspruch 1 worin in der Carbonylverbindung

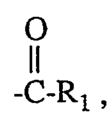

der Rest $R_1$ Wasserstoff oder Phenyl-, Alkyl- oder Cycloalkylrest mit 1 bis 6 Kohlenstoffatomen ist, der gegebenenfalls seinerseits mit Resten R" gleich Halogen, SR', $SO_3R'$, $NO_2$, NHR', $NR_2'$, NR'COR', OR', OCOR', CHO, $COR_1$, COOR', $CONR_2'$, CN oder Alkyl mit 1 bis 6 Kohlenstoffatomen substituiert ist, wobei R' Wasserstoff oder ein Phenyl-, Alkyl- oder Cycloalkylrest mit 1 bis 6 Kohlenstoffatomen bedeutet, oder $R_1$ ist gleich R", und/oder X und Y sind mit Resten $R_1$ substituiert.

3. Verbindung gemäss Anspruch 1 worin X ein Pyridin-, Pyridazin-, Pyrimidin-, Pyrazin-, Naphthalin-, Chinolin-, Benzothiazol-, Benzoxazol- oder Benzolrest ist, und Y, A und M die in Anspruch 1 genannte Bedeutung hat.

4. Verbindung gemäss Anspruch 1 worin Y die in Anspruch 3 für X genannte Bedeutung hat, und X, A und M die in Anspruch 1 genannte Bedeutung hat.

5. Verbindung gemäss Anspruch 1 worin A gleich CN ist und X, Y und M die in Anspruch 1 genannte Bedeutung hat.

6. Verbindung gemäss Anspruch 1 worin X und Y je die in Anspruch 3 unter X genannte Bedeutung hat, wobei X gleich Y oder ungleich Y sein kann, ohne dass X gleich Y gleich einem unsubstituierten Benzolrest ist, und A und M die in Anspruch 1 genannte Bedeutung hat.

7. Verbindung gemäss Anspruch 1 worin A gleich CN ist, X und Y die in Anspruch 6 genannte Bedeutung hat, und M gleich Lithium, Natrium, Kalium oder Ammonium ist.

8. Verbindung gemäss Anspruch 1 worin X und Y einen Benzolrest darstellen, , wobei mindestens einer der Benzolreste einfach oder mehrfach substituiert ist und A die in Anspruch 1 angegebene Bedeutung hat und M gleich Natrium ist.

9. Verbindung gemäss Anspruch 8 worin X gleich unsubstituiertes Phenylen, Y gleich substituiertes Phenyl und A gleich CN und M gleich Natrium ist.

10. Verbindung der Formel

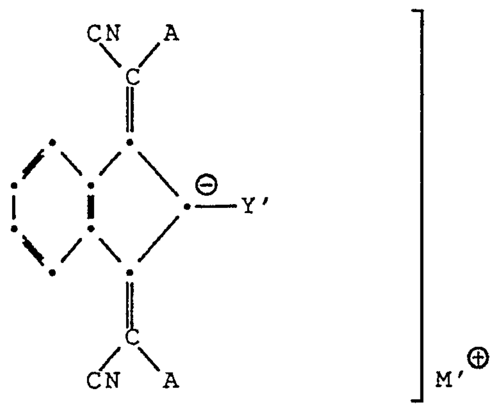

(Ia)

worin Y': H oder Phenyl, A: ein in Anspruch 1 genannter Rest und M' : Lithium, Natrium oder Kalium bedeutet.

11. Verbindung gemäss Anspruch 10, worin Y' Phenyl, A CN und M'$^{\oplus}$ Natrium ist.

12. Verfahren zur Herstellung der Verbindungen der Formeln I gemäss Anspruch 1 oder 10, dadurch gekennzeichnet, dass man Verbindungen der Formel

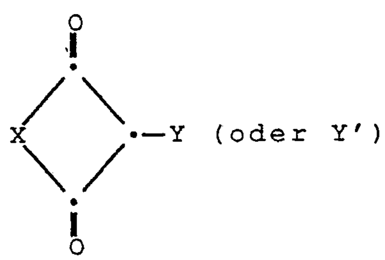

(II)

mit Verbindungen der Formel

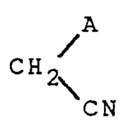

(III),

worin A, X und Y die in Anspruch 1 genannte Bedeutung haben und Y' H oder Phenyl bedeutet, in einem protischen Lösungsmittel oder in Gemischen von diesen, bei einer Temperatur von 10 °C bis zur Siedetemperatur des jeweiligen Lösungsmittels bzw. Gemisches und in Gegenwart eines sauren, basischen oder amphoteren Katalysators umsetzt, unter anschliessender Einführung des Kations M$^{\oplus}$ bzw. M'$^{\oplus}$ mittels Aufarbeitung.

**13.** Verwendung der Verbindung der Formel

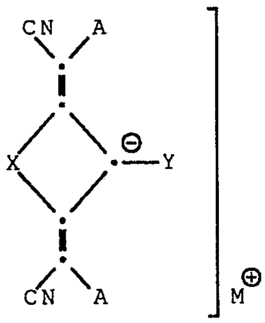

(Ic),

worin
X ein substituierter oder unsubstituierter, 5- oder 6-gliedriger monocyclischer oder polycyclischer aromatischer oder heteroaromatischer Rest ist,
Y Wasserstoff oder ein substituierter oder unsubstituierter, Alkenyl-, Alkyl- oder Cycloalkyl-Rest mit 1 bis 6 Kohlenstoffatomen oder ein 5- oder 6-gliedriger monocyclischer oder polycyclischer aromatischer oder heteroaromatischer Rest ist,
M ein Alkalimetall- oder Ammoniumion ist,
A, CN oder eine Carbonylverbindung

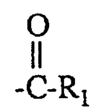

ist,
worin $R_1$ Wasserstoff oder ein Phenyl-, Alkyl- oder Cycloalkylrest mit 1 bis 6 Kohlenstoffatomen ist, der gegebenenfalls seinerseits mit Resten R" gleich Halogen, SR', $SO_3R'$, $NO_2$, NHR', $NR_2'$, NR'COR', OR', OCOR', CHO, $COR_1$, COOR', $CONR_2'$, CN oder Alkyl mit 1 bis 6 Kohlenstoffatomen substituiert ist, wobei R' Wasserstoff oder ein Phenyl-, Alkyl- oder Cycloalkylrest mit 1 bis 6 Kohlenstoffatomen bedeutet, oder $R_1$ ist gleich R", als Nuancierfarbstoff.

**14.** Verwendung gemäss Anspruch 13, dadurch gekennzeichnet, dass man eine Verbindung der Formel

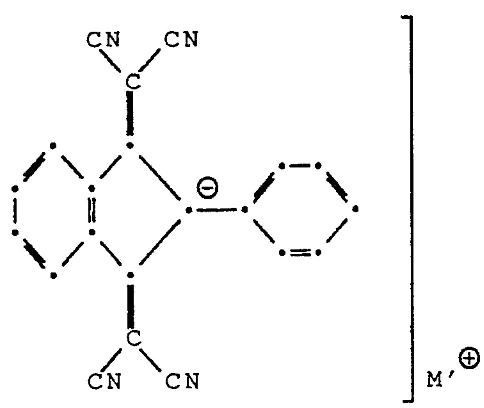

(Id)

worin $M'^{\oplus}$ Lithium, Natrium oder Kalium bedeutet, benutzt.

**15.** Verwendung gemäss Anspruch 14, dadurch gekennzeichnet, dass man das Natrium-Salz benutzt.

**16.** Verwendung gemäss Anspruch 13, dadurch gekennzeichnet, dass der Nuancierfarbstoff einem handelsüblichen Waschmittel in einer Menge von 0,0025-0,01 % bezogen auf die Waschmittelmenge zugesetzt

wird.

**17.** Verwendung gemäss Anspruch 16, dadurch gekennzeichnet, dass der Nuancierfarbstoff dem Waschmittel als ein Gemisch von Verbindungen gemäss Anspruch 13 und gegebenenfalls im Gemisch mit bekannten Nuancierfarbstoffen, Bleichmitteln, optischen Aufhellern und Lichtschutzmitteln zugesetzt wird.

**18.** Verwendung gemäss Anspruch 16, dadurch gekennzeichnet, dass das Waschgut Cellulose oder Cellulose/Polyester Mischmaterial enthält.

## Claims

**1.** An anionic cyclodiylidene compound of the formula

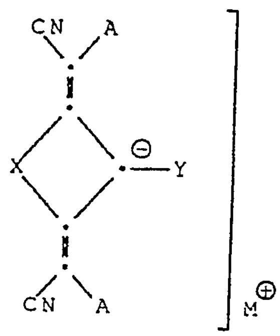

(I)

in which
X is a substituted or unsubstituted, 5- or 6-membered monocyclic or polycyclic aromatic or heteroaromatic radical, Y is hydrogen or a substituted or unsubstituted, alkenyl, alkyl or cycloalkyl radical having 1 to 6 carbon atoms or a 5- or 6-membered monocyclic or polycyclic aromatic or heteroaromatic radical, A is CN or a carbonyl compound, M is an alkali metal ion or ammonium ion, subject to the proviso that X is not unsubstituted phenylene when Y is hydrogen or unsubstituted phenyl.

**2.** A compound according to claim 1, in which, in the carbonyl compound

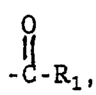

the radical $R_1$ is hydrogen or a phenyl, alkyl or cycloalkyl radical having 1 to 6 carbon atoms which in turn can be substituted by radicals R", equal to halogen, SR', $SO_3R'$, $NO_2$, NHR', $NR_2'$, NR'COR', OR', OCOR', CHO, $COR_1$, COOR', $CONR_2'$, CN or alkyl having 1 to 6 carbon atoms, where R' is hydrogen or a phenyl, alkyl or cycloalkyl radical having 1 to 6 carbon atoms, or $R_1$ is equal to R", and/or X and Y are substituted by radicals $R_1$.

**3.** A compound according to claim 1, in which X is a pyridine, pyridazine, pyrimidine, pyrazine, naphthalene, quinoline, benzothiazole, benzoxazole or benzene radical, and Y, A and M are as defined in claim 1.

**4.** A compound according to claim 1, in which Y is as defined for X in claim 3, and X, A and M are as defined in claim 1.

**5.** A compound according to claim 1, in which A is CN and X, Y and M are as defined in claim 1.

**6.** A compound according to claim 1, in which X and Y are each as defined for X in claim 3, it being

possible for X to be equal to Y or different from Y without X being identical to Y in being an unsubstituted benzene radical, and A and M are as defined in claim 1.

**7.** A compound according to claim 1, in which A is CN, X and Y are as defined in claim 6, and M is lithium, sodium, potassium or ammonium.

**8.** A compound according to claim 1, in which X and Y are a benzene radical, at least one of the benzene radicals being monosubstituted or polysubstituted, A is as defined in claim 1 and M is sodium.

**9.** A compound according to claim 8, in which X is unsubstituted phenylene, Y is substituted phenyl, A is CN and M is sodium.

**10.** A compound of the formula

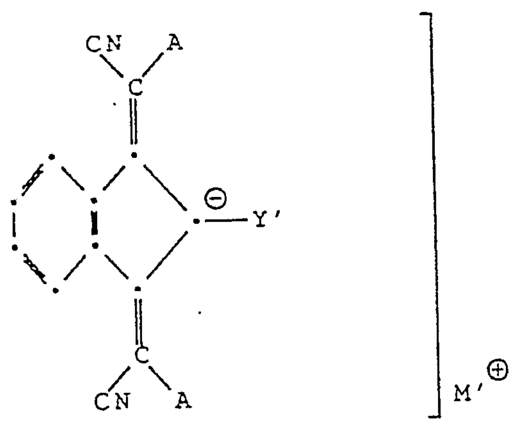

(Ia)

in which Y' is H or phenyl, A is a radical mentioned in claim 1 and M' is lithium, sodium or potassium.

**11.** A compound according to claim 10, in which Y' is phenyl, A is CN and $M'^{\oplus}$ is sodium.

**12.** A process for preparing a compound of the formula I according to claim 1 or 10, which comprises reacting a compound of the formula

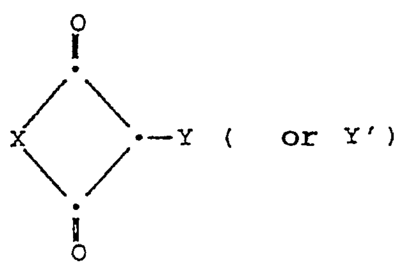

(II)

with a compound of the formula

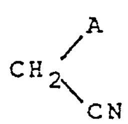

(III)

in which A, X and Y are as defined in claim 1 and Y' is H or phenyl in a protic solvent or in a mixture of such solvents at a temperature of 10 °C up to the boiling point of the particular solvent or mixture and in the presence of an acidic basic or amphoteric catalyst with subsequent introduction of the cation $M^{\oplus}$ or $M'^{\oplus}$ in the work-up.

**13.** Use of the compound of the formula

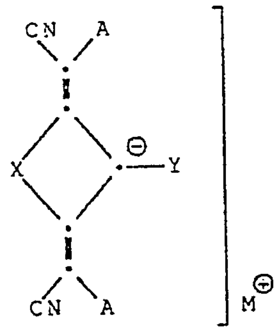

(Ic)

in which
X is a substituted or unsubstituted, 5- or 6-membered monocyclic or polycyclic aromatic or heteroaromatic radical, Y is hydrogen or a substituted or unsubstituted alkenyl, alkyl or cycloalkyl radical having 1 to 6 carbon atoms or a 5- or 6-membered monocyclic or polycyclic aromatic or heteroaromatic radical, M is an alkali metal or an ammonium ion, A is CN or a carbonyl compound

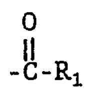

in which $R_1$ is hydrogen or a phenyl, alkyl or cycloalkyl radical having 1 to 6 carbon atoms which can in turn be further substituted by radicals R" equal to halogen, SR', $SO_3R'$, $NO_2$, NHR', $NR_2'$, NR'COR', OR', OCOR', CHO, $COR_1$, COOR', $CONR_2'$, CN or alkyl having 1 to 6 carbon atoms, where R' is hydrogen or a phenyl, alkyl or cycloalkyl radical having 1 to 6 carbon atoms, or $R_1$ is equal to R", as shading dye.

**14.** Use according to claim 13, wherein a compound of the formula

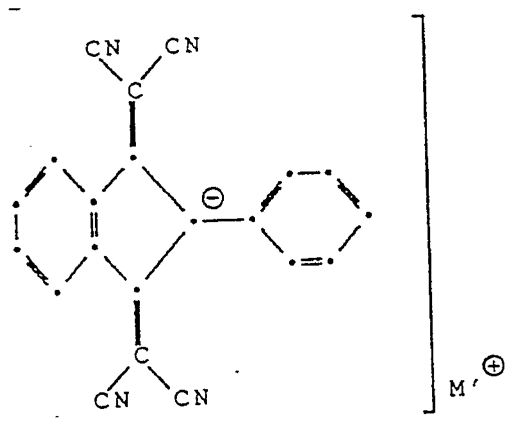

(Id)

in which $M'^{\oplus}$ is lithium, sodium or potassium, is used.

**15.** Use according to claim 14, wherein the sodium salt is used.

**16.** Use according to claim 13, wherein the shading dye is added to a commercial washing agent in an amount of 0.0025-0.01% based on the amount of washing agent.

**17.** Use according to claim 16, wherein the shading dye is added to the washing agent as a mixture of compounds according to claim 13 and if desired in a mixture with known shading dyes, bleaching agents, fluorescent whitening agents and light stabilizers.

**18.** Use according to claim 16, wherein the material to be washed contains cellulose or cellulose/polyester blend material.

**Revendications**

1. Composé cyclo-diylidène anionique, de formule

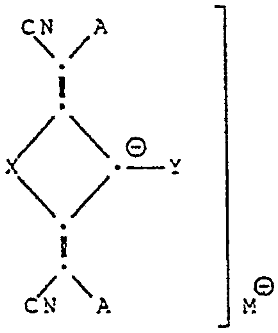

(I)

dans laquelle
X représente un résidu aromatique ou hétéro-aromatique, monocyclique à 5 ou 6 chaînons ou polycyclique, substitué ou non,
Y représente un atome d'hydrogène ou un groupe alcényle, alkyle ou cycloalkyle, substitué ou non, comportant de 1 à 6 atomes de carbone, ou bien un résidu aromatique ou hétéro-aromatique, monocyclique à 5 ou 6 chaînons ou polycyclique, A représente CN ou un résidu carbonylé, M représente un ion de métal alcalin ou un ion ammonium, à condition que X ne représente pas un résidu phénylène non substitué quand Y représente un atome d'hydrogène ou un groupe phényle non substitué.

2. Composé selon la revendication 1, dans lequel, dans le reste carbonylé -CO-$R_1$, le groupe $R_1$ représente un atome d'hydrogène ou un groupe phényle ou alkyle ou cycloalkyle comportant de 1 à 6 atomes de carbone, qui est éventuellement substitué à son tour par des groupes R", c'est-à-dire des atomes d'halogène ou des groupes SR', $SO_3R'$, $NO_2$, NHR', $NR'_2$, NR'COR', OR', OCOR', CHO, $COR_1$, COOR', $CONR'_2$, CN ou alkyle comportant de 1 à 6 atomes de carbone, R' représentant un atome d'hydrogène ou un groupe phényle ou alkyle ou cycloalkyle comportant de 1 à 6 atomes de carbone, ou encore $R_1$ représente R", et/ou X et Y sont substitués par des groupes $R_1$.

3. Composé selon la revendication 1, dans lequel X représente un reste de pyridine, pyridazine, pyrimidine, pyrazine, naphtalène, quinoline, benzothiazole, benzoxazole ou benzène, et Y, A et M ont les significations indiquées dans la revendication 1.

4. Composé selon la revendication 1, dans lequel Y a la signification indiquée dans la revendication 3 pour X, et X, A et M ont les significations indiquées dans la revendication 1.

5. Composé selon la revendication 1, dans lequel A représente -CN et X, Y et M ont les significations indiquées dans la revendication 1.

6. Composé selon la revendication 1, dans lequel X et Y ont chacun la signification indiquée pour X dans la revendication 3, X pouvant être identique ou non à Y, sans que X et Y représentent tous deux un reste de benzène non substitué, et A et M ont les significations indiquées dans la revendication 1.

7. Composé selon la revendication 1, dans lequel A représente -CN, X et Y ont les significations indiquées dans la revendication 6, et M représente lithium, sodium, potassium ou ammonium.

8. Composé selon la revendication 1, dans lequel X et Y représente un reste de benzène, au moins l'un des restes de benzène étant substitué une ou plusieurs fois, A a la signification indiquée dans la revendication 1 et M représente sodium.

9. Composé selon la revendication 8, dans lequel X représente un reste phénylène non substitué, Y représente un groupe phényle substitué, A représente -CN et M représente sodium.

**10.** Composé de formule

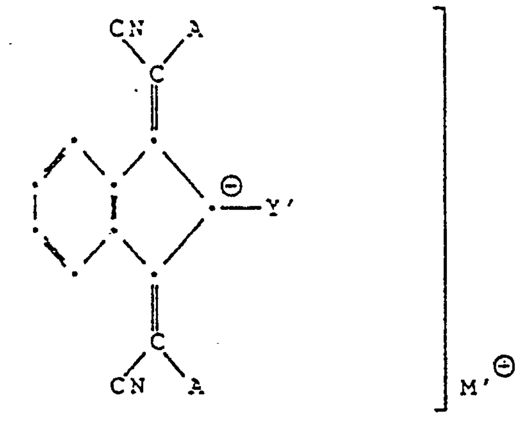

(Ia)

dans laquelle Y' représente H ou phényle, A représente un reste mentionné dans la revendication 1, et M' représente lithium, sodium ou potassium.

**11.** Composé selon la revendication 10, dans lequel Y' représente phényle, A représente -CN et $M'^{\oplus}$ représente sodium.

**12.** Procédé de préparation des composés de formule I selon la revendication 1 ou 10, caractérisé en ce que l'on fait réagir des composés de formule

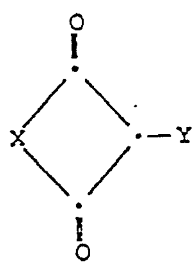

(ou Y')

(II)

avec des composés de formule

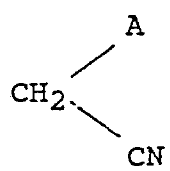

(III),

dans lesquelles A, X et Y ont les significations indiquées dans la revendication 1 et Y' représente H ou phényle, dans un solvant protique ou dans des mélanges de tels solvants, à une température située entre 10 °C et la température d'ébullition du solvant ou du mélange de solvants, et en présence d'un catalyseur acide, basique ou amphotère, avec introduction subséquente du cation $M^{\oplus}$ ou $M'^{\oplus}$ par traitement ultérieur.

**13.** Utilisation du composé de formule

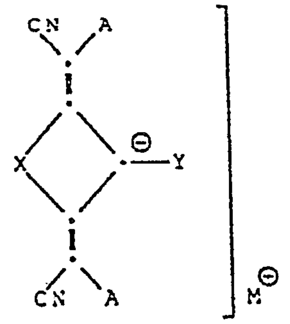

(Ic),

dans laquelle
X représente un résidu hétéro-aromatique ou aromatique, monocyclique à 5 ou 6 chaînons ou polycyclique, substitué ou non,
Y représente un atome d'hydrogène ou un groupe alcényle, alkyle ou cycloalkyle, substitué ou non, comportant de 1 à 6 atomes de carbone, ou bien un résidu aromatique ou hétéro-aromatique, monocyclique à 5 ou 6 chaînons ou polycyclique,
M représente un ion de métal alcalin ou un ion ammonium,
A représente CN ou un reste carbonylé -CO-$R_1$, dans lequel $R_1$ représente un atome d'hydrogène ou un groupe phényle ou un groupe alkyle ou cycloalkyle comportant de 1 à 6 atomes de carbone, qui est lui-même éventuellement substitué par des groupes R", c'est-à-dire halogène, SR', $SO_3R'$, $NO_2$, NHR', $NR'_2$, NR'COR', OR', OCOR', CHO, $COR_1$, COOR', $CONR'_2$, CN ou alkyle comportant de 1 à 6 atomes de carbone, R' représentant un atome d'hydrogène ou un groupe phényle ou alkyle ou cycloalkyle comportant de 1 à 6 atomes de carbone, ou encore $R_1$ est identique à R",
en tant que colorants de nuançage.

**14.** Utilisation selon la revendication 13, *caractérisée* en ce que l'on se sert d'un composé de formule

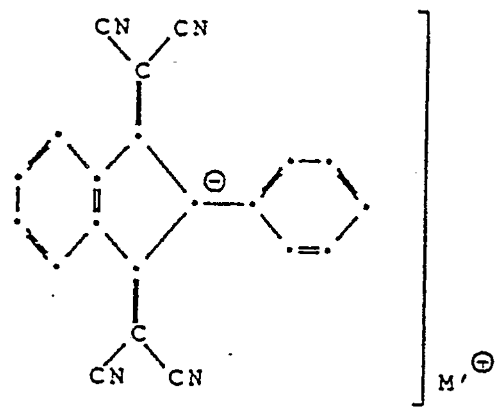

(Id)

dans laquelle M' ⊕ représente lithium, sodium ou potassium.

**15.** Utilisation selon la revendication 14, *caractérisée* en ce que l'on se sert du sel de sodium.

**16.** Utilisation selon la revendication 13, *caractérisée* en ce que l'on ajoute le colorant de nuançage à un détergent commercial, en une proportion de 0,0025 à 0,01 % par rapport à la quantité de détergent.

**17.** Utilisation selon la revendication 16, *caractérisée* en ce que le colorant de nuançage est ajouté au détergent sous la forme d'un mélange de composés selon la revendication 13, et éventuellement, mélangé avec des colorants de nuançage, agents de blanchiment, azurants optiques et agents de protection contre la lumière connus.

**18.** Utilisation selon la revendication 16, *caractérisée* en ce que la matière à laver contient de la cellulose ou un matériau mélangé cellulose/polyester.